# EUROPÄISCHE PATENTSCHRIFT

(11) **EP 3 553 499 B1**
(45) Veröffentlichungstag und Bekanntmachung des Hinweises auf die Patenterteilung: **25.03.2020**
(21) Anmeldenummer: 18167338.5
(22) Anmeldetag: 13.04.2018
(51) Int. Cl.: G01N 21/33, B01D 53/00, G01N 31/00, G01N 33/00, F01N 3/00, F01N 11/00, B01D 53/30, B01D 53/56

(54) **GASANALYSATOR UND VERFAHREN ZUR MESSUNG VON STICKOXIDEN IN EINEM ABGAS**
GAS ANALYSIS SYSTEM AND METHOD FOR MEASURING NITROGEN OXIDES IN A WASTE GAS
ANALYSEUR DE GAZ ET PROCÉDÉ DE MESURE DES OXYDES D'AZOTE DANS UN GAZ D'ÉCHAPPEMENT

(43) Veröffentlichungstag der Anmeldung: 16.10.2019
(73) Patentinhaber: Siemens Aktiengesellschaft, 80333 München (DE)
(72) Erfinder: HEFFELS, Camiel, 76297 Stutensee-Büchig (DE); ROßFELD, Daniel, 66907 Glan-Münchweiler (DE)

(56) Entgegenhaltungen:
- WO-A1-2015/181879
- JP-A- S60 119 443
- JP-A- 2012 026 331
- US-A1- 2015 153 272

## Beschreibung

Die Erfindung betrifft einen Gasanalysator zur Messung von Stickoxiden in einem Abgas mit
- einer Oxidationseinrichtung, die einen Ozongenerator zur Erzeugung von Ozon aus Sauerstoff aufweist und zur Behandlung des Abgases mit dem Ozon ausgebildet ist, um in dem Abgas enthaltenes Stickstoffmonoxid in Stickstoffdioxid umzusetzen,
- einer mit einer Zentralwellenlänge zwischen 350 nm und 500 nm strahlenden ersten Leuchtdiode,
- einer mit einer Zentralwellenlänge zwischen 250 nm und 265 nm strahlenden zweiten Leuchtdiode,
- einer von dem behandelten Abgas durchströmten und dem Licht der ersten Leuchtdiode und dem Licht der zweiten Leuchtdiode durchstrahlten Messkammer,
- einem das Licht der ersten Leuchtdiode und das Licht der zweiten Leuchtdiode nach Durchstrahlen der Messkammer detektierenden Detektor, welcher ein Detektorsignal mit einem aus dem Licht der ersten Leuchtdiode resultierenden ersten Signalanteil und einen aus dem Licht der zweiten Leuchtdiode resultierenden zweiten Signalanteil erzeugt, und
- einer Auswerteeinrichtung, die aus dem ersten Signalanteil die Stickstoffdioxid-Konzentration des behandelten Abgases in der Messkammer ermittelt und als die Stickoxid-Konzentration des Abgases ausgibt aus dem zweiten Signalanteil die Ozon-Konzentration des Abgases in der Messkammer ermittelt.

Die Erfindung betrifft ferner ein Verfahren zur Messung von Stickoxiden in einem Abgas wobei
- mittels eines Ozongenerators aus Sauerstoff Ozon erzeugt wird,
- das Abgas mit dem erzeugten Ozon behandelt wird, um in dem Abgas enthaltenes Stickstoffmonoxid in Stickstoffdioxid umzusetzen,
- die Stickstoffdioxid-Konzentration des behandelten Abgases photometrisch unter Verwendung einer mit einer Zentralwellenlänge zwischen 350 nm und 500 nm strahlenden ersten Leuchtdiode gemessen und als die Stickoxid-Konzentration des Abgases ausgegeben wird und
- die Ozon-Konzentration des behandelten Abgases photometrisch unter Verwendung einer mit einer Zentralwellenlänge zwischen 250 nm und 265 nm strahlenden zweiten Leuchtdiode gemessen wird,
- wobei das Licht der ersten Leuchtdiode und der zweiten Leuchtdiode eine mit dem behandelten Abgas durchströmte Messkammer durchstrahlt.

Ein derartiger Gasanalysator und ein derartiges Verfahren sind aus der WO 2015/181879 A1 bekannt.

Aus Ryoichi Higashi et al.: "A NOx and SO2 gas analyzer using deep-UV and violet light-emitting diodes for continuous emissions monitoring systems", Proc. SPIE 9003, Light-Emitting Diodes: Materials, Devices, and Applications for Solid State Lighting XVIII, 90031F (February 27, 2014), ist ein Gasanalysator zur Messung von Stickoxiden (NOx) und Schwefeldioxid (SO2) in einem Abgas bekannt. Vor der Analyse wird das Abgas in zwei Stufen behandelt, wobei in einer ersten Stufe in dem Abgas enthaltenes Stickstoffmonoxid (NO) mit Hilfe von Ozon (O3) in mit dem Gasanalysator messbares Stickstoffdioxid (NO2) umgesetzt wird. Das Ozon wird mittels elektrischer Entladung aus Luftsauerstoff erzeugt und dem Abgas zugeführt. In einer zweiten Behandlungsstufe wird das Abgas auf etwa 300 °C erhitzt, um überschüssiges Ozon und durch Reaktion von Stickstoffdioxid und Ozon entstandenes und von dem Gasanalysator nicht messbares Distickstoffpentoxid (N2O5) thermisch in Stickstoffdioxid zu zersetzen. Die von dem Gasanalysator ermittelte Konzentration von Stickstoffdioxid ist somit ein Maß für die Konzentration von Stickoxiden in dem Abgas.

Für eine möglichst vollständige Umsetzung von Stickstoffmonoxid in Stickstoffdioxid muss das Ozon mit einem gewissen Überschuss erzeugt werden. Bei der anschließenden thermischen Zersetzung von Distickstoffpentoxid kann jedoch neben Stickstoffdioxid auch wieder unerwünschtes Stickstoffmonoxid entstehen, weswegen die Aufheizung des Abgases kontrolliert erfolgen sollte. Dabei oder aufgrund von Störungen kann auch die thermische Zersetzung des überschüssigen Ozons unvollständig erfolgen.

Bei dem bekannten Gasanalysator sind eine Leuchtdiode mit einer Emissionswellenläge von 280 nm und eine Leuchtdiode mit einer Emissionswellenläge von 400 nm dicht nebeneinander in einem LED-Array angeordnet. Ihr Licht wird mittels einer Kollimatorlinse zu einem parallelen Lichtbündel geformt, das eine von dem behandelten Abgas durchströmte Messkammer durchstrahlt und anschließend auf einen Detektor fokussiert wird. Mittels eines Strahlteilers zwischen Kollimatorlinse und Messkammer wird ein Teil des Lichts auf einen Monitordetektor gelenkt. Die Leuchtdioden werden im Wechsel ein- und ausgeschaltet, um das in dem Abgas enthaltene Schwefeldioxid bei der Absorptionswellenlänge 280 nm und Stickstoffdioxid bei der Absorptionswellenlänge 400 nm zu detektieren. Das Detektorsignal wird mit dem Signal des Monitordetektors normalisiert, bevor es zur Ermittlung der Schwefeldioxid- und Stickstoffdioxid- bzw. Stickoxid-Konzentrationen in dem Abgas ausgewertet wird. Die Temperatur der Leuchtdioden wird mittels eines Peltierelements auf einen konstanten Wert geregelt.

Bei dem aus der oben genannten WO 2015/181879 A1 bekannten Gasanalysator wird das Ozon zur Behandlung des Abgases aus Umgebungsluft durch stille elektrische Entladung erzeugt und dem Abgas beigemischt. Die Ozonerzeugung bzw. der Ozongenerator werden durch die Auswerteeinrichtung des Gasanalysators ein- und ausgeschaltet. Bei eingeschaltetem Ozongenerator werden die Stickstoffdioxid-Konzentration des behandelten Abgases photometrisch bei einer Zentralwellenlänge zwischen 350 nm und 500 nm und die Ozonkonzentration bei einer Zentralwellenlänge zwischen 240 nm und 330 nm ermittelt. Bei ausgeschaltetem Ozongenerator wird die Stickstoffdioxid-Konzentration des unbehandelten Abgases ermittelt. Anhand der bei sowohl eingeschaltetem als auch ausgeschaltetem Ozongenerator ermittelten Konzentrationen und unter Verwendung eines entweder bekannten oder mittels eines Ozonmessers in dem Ozongenerator gemessenen Wertes für das erzeugte Ozon wird die Stickstoffmonoxid-Konzentration des Abgases rechnerisch ermittelt. Die Zentralwellenlänge zwischen 240 nm und 330 nm, insbesondere bei 280 nm, kann dazu verwendet werden, bei ausgeschaltetem Ozongenerator Schwefeldioxid-Konzentration in dem Abgas zu ermitteln.

Aus der WO 2016/112943 A1 ist ebenfalls eine Anordnung zur Umsetzung von mit derzeitigen Mitteln nicht direkt messbarem Stickstoffmonoxid mittels Ozon in messbares Stickstoffdioxid bekannt. Das Ozon wird in einem Ozongenerator aus Umgebungsluft mit Hilfe von UV-Licht, beispielsweise einer Quecksilberdampflampe bei einer Wellenlänge von 184,9 nm, erzeugt und anschließend in einer Oxidationseinheit dem Messgas (hier: Messluft) zur Oxidation des darin enthaltenen Stickstoffmonoxids beigemischt. Auch hier besteht das Problem, dass insbesondere bei hoher Ozonerzeugungsrate neben Ozon auch unerwünschte höhere Stickoxide erzeugt werden, während bei niedriger Ozonerzeugungsrate die Ozonkonzentration zu gering ist, um einerseits eine schnelle und vollständige Umsetzung von Stickstoffmonoxid in Stickstoffdioxid zu bewirken und andererseits die Verdünnung der Messluft möglichst gering zu halten. Aus diesem Grund wird daher der Oxidationseinheit ein Silicagel-Filter nachgeordnet, um höhere Stickoxide wie Stickstoffdioxid, Stickstofftrioxid (NO3) und Distickstoffpentoxid herauszufiltern bzw. zu absorbieren.

Aus der EP 2 157 421 A1 ist es bekannt, zur Untersuchung des Schwefelgehalts von Kraftstoffen eine Probe des Kraftstoffs zu verbrennen und die Konzentration von Schwefeldioxid in dem Abgas durch ein Ultraviolett-Fluoreszenz-Messverfahren zu bestimmen. Um die Beeinträchtigung der Messung durch Stickstoffmonoxid zu vermeiden, wird das Abgas zuvor in einem Behälter dem Licht (185 nm) einer Niederdruck-Quecksilberentladungslampe ausgesetzt, um aus dem Restsauerstoffgehalt Ozon zu erzeugen und damit Stickstoffmonoxid in Stickstoffdioxid umzusetzen.

Aus der EP 0 697 374 A1 ist eine Durchfluss-Excimerlampe zum Behandeln von Fluiden (Gas oder Flüssigkeiten) mit UV-Strahlung bekannt. Ein typischer Excimer-Strahler weist ein zylindrisches, doppelwandiges Quarzrohr auf, das in dem Ringraum zwischen den Wänden mit einem Edelgas, z. B. Xenon, gefüllt ist. Elektroden, die sich außen auf der inneren und äußeren Wand des Quarzrohrs befinden, sind mit einem Hochfrequenz-Generator (HF-Generator) verbunden. Aufgrund der hochfrequenten Hochspannung induzieren Elektroden im Entladungsraum Mikroentladungen, durch die Excimermoleküle gebildet werden. Die Excimermoleküle zerfallen kurz nach ihrer Erzeugung wieder, wobei im Falle von Xenon jeweils ein 172 nm UV-Photon emittiert wird. Bei der bekannten Durchfluss-Excimerlampe dient das innere Rohr als Durchflussstrecke für das zu behandelnde Fluid. Die innere Elektrode ist als Drahtwendel ausgebildet, der mit dem Fluid in Kontakt steht, während die äußere Elektrode das doppelwandige Quarzrohr umhüllt und als Reflektor für die UV-Strahlung wirkt. Um bei der UV-Behandlung von Flüssigkeiten einen guten Wirkungsgrad zu erzielen, kann die innere Elektrode als statischer Mischer für das Fluid ausgebildet sein. Aufgrund der Mischwirkung wird eine Entmischung verhindert, so dass beispielsweise bei der Behandlung von belastetem Wasser Luft- oder Sauerstoffblasen mitgeführt werden können. Eine Sauerstoffanreicherung im Wasser kann den Abbauprozess von Schadstoffen erheblich beschleunigen. Auch lässt sich eine durch die UV-Strahlung hervorgerufene Ozonbildung erhalten.

Aus der US 2010/061885 A1 ist ein Gasanalysator zur Messung von Ozon in einem Gasgemisch, z. B. Luft, bekannt, bei dem eine das Gasgemisch enthaltende Messkammer mit dem Licht einer Leuchtdiode bei einer Zentralwellenlänge zwischen 250 nm und 290 nm, insbesondere bei 265 nm, durchstrahlt wird. Das Licht wird anschließend detektiert und zur Ermittlung der Ozon-Konzentration ausgewertet.

Aus der JP 2012 026331 A ist es bekannt, das Abgas in einem Kraftfahrzeug mit Ozon zu behandeln, wobei ein Stickstoffmonoxid-Stickstoffdioxid-Verhältnis 1:1 angestrebt wird, um die Stickoxid- Reduktion in einem nachgeordneten SCR-Katalysator zu optimieren. Damit kein überschüssiges Ozon in die Umgebung gelangt, ist im Abgasweg vor dem Auspuff des Kraftfahrzeugs ein Halbleitersensor angeordnet, mit dessen Signal die Ozonerzeugung geregelt wird. Das Ozon wird mittels Hochspannung aus Umgebungsluft erzeugt und dem Abgas beigemischt.

Aus der US 2015/153272 A1 ist ein Absorptionsphotometer mit zwei der mehr UV-LEDs zur Konzentrationsbestimmung von Proteinen, Peptide, Nukleinsäure usw. in Lösungen bekannt.

Aus der JP S60 119443 A ist ein nichtdispersiver Infrarot-(NDIR-) Zweistrahl-Gasanalysator mit einer Messzelle, einer dazu parallelen Referenzzelle und einem optopneumatischen Detektor mit zwei nebeneinander liegenden Empfängerkammern bekannt. Zur Messung von Stickstoffmonoxid in einem Messgas (Abgas) wird dieses zuerst durch die Messzelle und danach über eine Oxidationseinrichtung durch die Referenzzelle geleitet. In der Oxidationseinrichtung wird durch stille elektrische Entladung oder mittels UV-Licht in dem Abgas enthaltener Sauerstoff in Ozon umgesetzt, mit dem wiederum das Stickstoffmonoxid vollständig in Stickstoffdioxid umgesetzt wird. Die beiden Empfängerkammern sind mit Stickstoffmonoxid gefüllt und daher nur für diese Gaskomponente selektiv, so dass der Zweistrahl-Gasanalysator die Stickstoffmonoxid-Konzentration des Messgases von weiteren Begleitgasen unbeeinflusst misst.

Der Erfindung liegt die Aufgabe zugrunde, eine zuverlässige kontinuierliche Messung von Stickoxiden und in Abgasen mit geringem gerätetechnischem Aufwand zu ermöglichen.

Gemäß der Erfindung wird die Aufgabe dadurch gelöst, dass bei dem Gasanalysator der eingangs angegebenen Art
- der Ozongenerator regelbar ist, und
- eine den Ozongenerator steuernde Regeleinrichtung vorhanden ist, die die ermittelte Ozon-Konzentration als Istwert erhält, um sie auf einen vorgegebenen Sollwert zu regeln.

In Bezug auf das eingangs angegebene Verfahren wird die Aufgabe dadurch gelöst,
- dass der Ozongenerator regelbar ist und mit einer Regeleinrichtung gesteuert wird, und
- dass die Regeleinrichtung die gemessene Ozon-Konzentration als Ist-Wert erhält und sie auf einen vorgegebenen Sollwert regelt.

Die Regelung des Ozongenerators bzw. der Erzeugung des Ozons mit den in dem behandelten Abgas gemessenen Ozon-Konzentrationswerten stellt sicher, dass nur so viel Ozon produziert wird, wie für die Umsetzung von Stickstoffmonoxid in Stickstoffdioxid erforderlich ist. Da folglich kein oder nur wenig überschüssiges Ozon produziert wird, ist die Umsetzung von Stickstoffmonoxid zu Stickstoffdioxid nahezu vollständig, wodurch eine lineare Messung des Stickstoffmonoxids mit dem kostengünstigen LED-Stickstoffdioxid-Gasanalysators realisiert wird.

Die erste und zweite Leuchtdiode können in an sich bekannter Weise dicht nebeneinander in einem LED-Array angeordnet sein, oder ihr Licht kann mittels eines Strahlteilers zu einem Lichtbündel durch die Messkammer vereinigt werden. Zur Referenzierung (Normierung) der Messungen ist der (oder ein) Strahlteiler vorzugsweise dazu ausgebildet, einen Teil des Lichts der beiden Leuchtdioden auf einen Referenzdetektor abzuzweigen, der ein Referenzsignal mit aus dem Licht der ersten und zweiten Leuchtdiode resultierenden ersten und zweiten Referenz-Signalanteilen erzeugt. In der Auswerteeinrichtung findet dann die Referenzierung der beiden Signalanteile des Detektorsignals mit den zugehörigen Referenz-Signalanteilen statt, beispielsweise durch Quotientenbildung. Um die unterschiedlichen Signalanteile des Detektorsignals und des Referenzsignals unterscheiden zu können, können die Leuchtdioden im Zeitmultiplex nacheinander angesteuert werden oder ihr Licht kann bei der Erzeugung unterschiedlich moduliert und bei der Detektion z. B. phasensensitiv demoduliert werden.

Die Ozonerzeugung in der Oxidationseinrichtung kann auf unterschiedliche Wese erfolgen. Die in der Publikation von Ryoichi Higashi et al. erwähnte Ozonerzeugung aus Luftsauerstoff mittels elektrischer Entladung (Koronaentladung) hat jedoch den Nachteil, dass sich Stickoxidverbindungen bilden, die für die Stickoxid-Messung des Abgases unerwünscht sind. Die Oxidationseinrichtung des erfindungsgemäßen Gasanalysators weist daher vorzugsweise einen Ozongenerator mit mindestens einer Ultraviolett-Lichtquelle auf, die hochenergetische Strahlung mit einer Wellenlänge kleiner als 240 nm erzeugt (Wellenlängenanteile größer als 240 nm führen zu einer Zerstörung des zu gebildeten Ozons). Grundsätzlich geeignete Ultraviolett-Lichtquellen sind Niederdruck-Quecksilberentladungslampen oder elektrodenlose Excimerlampen. Vorzugsweise weist die Ultraviolett-Lichtquelle mindestens einen Xenon-Excimer-Strahler auf, der sich durch eine hohe Lebensdauer, geringe Temperaturabhängigkeit, einfache Dimmbarkeit und den völlig Verzicht auf Quecksilber auszeichnet.

Bei magerer Verbrennung mit Luftüberschuss kann das Ozon in vorteilhafter Weise vollständig aus dem Restsauerstoffgehalt des Abgases erzeugt werden. In diesem Fall ist die Ultraviolett-Lichtquelle in einer von dem Abgas durchströmten geschlossenen Reaktionskammer angeordnet. Die Reaktionskammer kann z. B. in Form eines innenverspiegelten Rohres (z. B. Aluminium-Rohr) ausgebildet sein, in dem sich die beispielsweise zylinderförmige Ultraviolett-Lichtquelle erstreckt. Zur Erhöhung der Leistung bei der Umsetzung von Stickstoffmonoxid in Stickstoffdioxid können mehrerer solcher Rohre parallel geschaltet sein. Alternativ kann mindestens eine Durchfluss-Excimerlampe verwendet werden, wie sie an sich aus der oben erwähnten EP 0 697 374 A1 bekannt ist.

Wenn die Leistung der Ultraviolett-Lichtquelle mit der Zeit nachlässt, kann dem Abgas zusätzlich Luftsauerstoff zugeführt werden. Es ist auch möglich, das Ozon aus dem Luftsauerstoff zu erzeugen und dem zu behandelnden Abgas beizumischen.

Trotz des mittlerweile verbreiteten Einsatzes schwefelarmer Brennstoffe besteht in vielen Fällen das Erfordernis, bei Analyse von Abgase auch Schwefeldioxid zu messen. Zu diesem Zweck kann der erfindungsgemäße Gasanalysator eine mit einer Zentralwellenlänge zwischen 250 nm und 300 nm strahlende dritte Leuchtdiode aufweisen, deren Licht ebenfalls die Messkammer durchstrahlt und anschließend von dem Detektor detektiert wird. Das Detektorsignal enthält daher einen dritten Signalanteil, aus dem die Auswerteeinrichtung die Schwefeldioxid-Konzentration des Abgases ermittelt. Auch hier können die erste und zweite Leuchtdiode oder alle drei Leuchtdioden dicht nebeneinander in einem LED-Array angeordnet sein, oder ihr Licht kann mittels mindestens eines Strahlteilers zu einem Lichtbündel durch die Messkammer vereinigt werden. Ferner kann auch hier zur Referenzierung der Messungen ein Teil des Lichts der drei Leuchtdioden auf einen Referenzdetektor abgezweigt werden.

Die Querempfindlichkeit der Restkonzentration des Ozons in dem behandelten Abgas auf die Messung der Schwefeldioxid-Konzentration in dem oben erwähnten Wellenlängenbereich von 250 nm und 300 nm kann mit der gemessenen Ozon-Restkonzentration rechnerisch kompensiert werden.

Alternativ kann die Schwefeldioxid-Konzentration in dem unbehandelten Abgas gemessen werden. Darüber hinaus kann in vorteilhafter Weise auch die Stickstoffdioxid-Konzentration in dem unbehandelten Abgas gemessen werden, so dass aus der Differenz der in dem behandelten Abgas und in dem unbehandelten Abgas ermittelten Stickstoffdioxid-Konzentrationen die Stickstoffmonoxid-Konzentration des Abgases ermittelt werden kann.

Wenn das Ozon nicht ausschließlich aus dem Restsauerstoffgehalt des Abgases erzeugt wird, kann beispielsweise der zusätzliche zugeführte Sauerstoff (z. B. Luftsauerstoff) mit dem Abgas zunächst durch die erste Messkammer geführt und dort gemessen werden, bevor er in die Oxidationseinrichtung gelangt.

Weitere vorteilhafte Ausgestaltungen des erfindungsgemäßen Gasanalysators und Verfahrens ergeben sich aus den Unteransprüchen und/oder den folgenden Ausführungsbeispielen.

Zur weiteren Erläuterung der Erfindung wird im Folgenden auf die Figuren der Zeichnung Bezug genommen; im Einzelnen zeigen
- Figur 1: ein erstes Ausführungsbeispiel des erfindungsgemäßen Gasanalysators zur Messung von Stickoxiden,
- Figur 2: ein Ausführungsbeispiel des Ozongenerators,
- Figur 3: beispielhaft die Absorptionsspektren von Stickstoffdioxid, Ozon und Schwefeldioxid sowie die Emissionsspektren der Leuchtdioden des Gasanalysators,
- Figur 4: ein zweites Ausführungsbeispiel des erfindungsgemäßen Gasanalysators zur Messung von Stickoxiden und Schwefeldioxid,
- Figur 5: ein drittes Ausführungsbeispiel des erfindungsgemäßen Gasanalysators zur Messung von Stickoxiden und Schwefeldioxid,
- Figur 6: ein viertes Ausführungsbeispiel des erfindungsgemäßen Gasanalysators zur Messung von Stickoxiden und Schwefeldioxid.

Gleiche und gleichwirkende Teile sind mit jeweils denselben Bezugszeichen versehen.

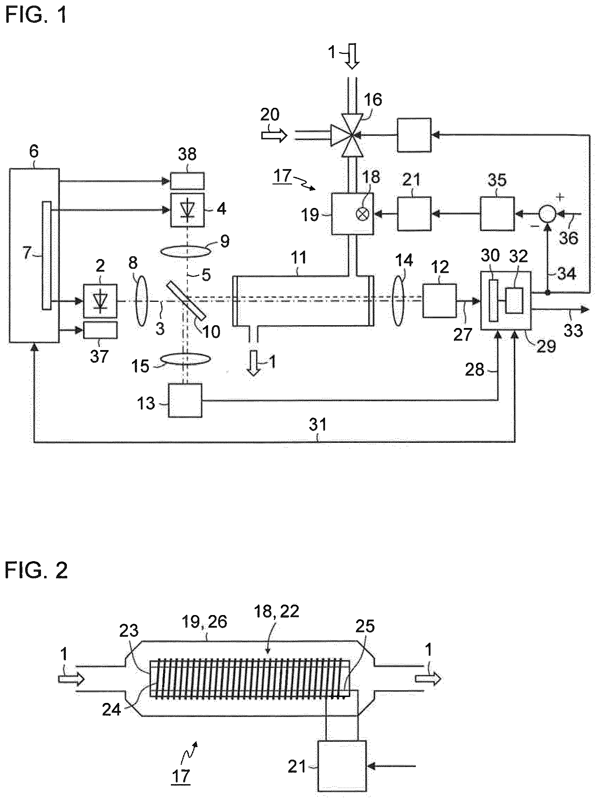

Figur 1 zeigt in vereinfachter schematischer Darstellung ein Blockschaltbild eines Gasanalysators zur Messung von Stickoxiden in einem Abgas 1. Eine erste Leuchtdiode 2 erzeugt Licht 3 im Wellenlängenbereich zwischen 350 nm und 500 nm, beispielsweise mit einer Zentralwellenlänge von 405 nm. Eine zweite Leuchtdiode 4 erzeugt Licht 5 im Wellenlängenbereich zwischen 250 nm und 265 nm, beispielsweise mit einer Zentralwellenlänge von 254 nm. Beide Leuchtdioden 2, 4 werden von einer Steuereinrichtung 6 angesteuert, die bei dem hier gezeigten Ausführungsbeispiel einen Multiplexer 7 enthält, um die Leuchtdioden 2, 4 abwechselnd ein- und auszuschalten. Das von den Leuchtdioden 2, 4 abgestrahlte Licht 3, 5 wird mit Hilfe von Kollimatorlinsen 8, 9 zu parallelen Lichtbündeln (im Weiteren als Lichtstrahlen bezeichnet) geformt und einem Strahlteiler 10 zugeführt, vorzugsweise einem sog. Polka-Dot-Strahlteiler, der ein konstantes Reflexions-/Transmissionsverhältnis über einen großen Spektralbereich aufweist. Der Strahlteiler 10 teilt das Licht 3, 5 der Leuchtdioden 2, 4 in einen Teilstrahl durch eine Messkammer 11 hindurch auf einen Detektor 12 und einen weiteren Teilstrahl auf einen Referenzdetektor 13 auf. Die jeweiligen Teilstrahlen werden mit Hilfe von Linsen 14, 15 auf die Detektoren 12, 13 fokussiert, bei denen es sich hier um Photodioden handelt.

Das Abgas 1 wird nach einer hier nicht gezeigten Vorbehandlung, die eine Entfeuchtung mittels Gaskühlung und Kondensatabscheidung sowie eine Partikelfeinfilterung umfasst, über ein optionales steuerbares Dreiwege-Mischventil 16 zunächst durch eine Oxidationseinrichtung 17 geleitet, bevor es in die Messkammer 11 gelangt. Der Abgasstrom kann in an sich bekannter Weise durch Druck- oder Strömungsregelung auf vorgegebene Durchflusswerte eingestellt und konstant gehalten werden. Nach Durchströmen der Messkammer 11 wird das Abgas 1 aus dieser ausgeleitet.

Die Oxidationseinrichtung 17 weist einen Ozongenerator 18 in Form einer Ultraviolett-Lichtquelle in einer von dem Abgas 1 durchströmten geschlossenen Reaktionskammer 19 auf. Die Ultraviolett-Lichtquelle 18 erzeugt mit ihrer UV-Strahlung bei Wellenlängen kleiner als 240 nm aus dem Restsauerstoff des Abgases 1 Ozon, mit dem das in dem Abgas 1 enthaltene Stickstoffmonoxid in der Reaktionskammer 19 zu Stickstoffdioxid und einigen höheren Stickoxiden, hauptsächlich Stickstofftrioxid und Distickstoffpentoxid reagiert. Allerdings entstehen die höheren Stickoxide nur dann, wenn zu viel Ozon in dem Ozongenerator 18 produziert wird. Wenn dagegen kein oder nur wenig überschüssiges Ozon produziert wird, findet eine nahezu vollständige Umsetzung von Stickstoffmonoxid in Stickstoffdioxid statt.

Das steuerbare Dreiwege-Mischventil 16 dient dazu, dem Abgas 1 Luftsauerstoff 20 beizumischen, wenn der Restsauerstoffgehalt des Abgases 1 oder die Leistung des Ozongenerators 18 nicht ausreichen, die für die vollständige Umsetzung von Stickstoffmonoxid in Stickstoffdioxid erforderliche Ozonmenge zu erzeugen. Die Ultraviolett-Lichtquelle 18 ist dimmbar und wird von einem Betriebsgerät 21 versorgt bzw. angesteuert.

Figur 2 zeigt sehr vereinfacht ein Ausführungsbeispiel der Oxidationseinrichtung 17 mit hier einer Ultraviolett-Lichtquelle 18 in Form eines handelsüblichen Xenon-Excimer-Strahlers 22. Dieser weist in bekannter Weise ein zylindrisches, doppelwandiges Quarzrohr 23 auf, das in dem Ringraum zwischen den Wänden mit Xenon gefüllt ist. Auf der inneren und äußeren Wand des Quarzrohres 23 angeordnete Elektroden 24, 25 sind an dem Betriebsgerät 21 angeschlossen. Die Reaktionskammer 19 wird von einen innenverspiegelten Aluminium-Rohr 26 gebildet, in dem der Xenon-Excimer-Strahler 22 angeordnet ist und das von dem Abgas 1 durchströmt wird. Der Xenon-Excimer-Strahler 22 erzeugt eine UV-Strahlung mit einer Hauptwellenlänge von 172 nm, mittels derer aus dem Restsauerstoff des Abgases 1 Ozon erzeugt wird, mit dem das in dem Abgas 1 enthaltenes Stickstoffmonoxid in Stickstoffdioxid umsetzt wird. Bei vergleichsweise geringe Stickstoffmonoxid-Konzentration des Abgases 1 (z. B. < 5 ppm) kann es von Vorteil sein, die Intensität der abgegebenen UV-Strahlung von vorneherein zu drosseln, was bei dem gezeigten Ausführungsbeispiel dadurch geschieht, dass die äußere Elektrode 24 ist von einem Draht gebildet ist, der auf dem Quarzrohr 23 aufgewickelt ist.

Alternativ zu dem gezeigten Ausführungsbeispiel kann die Ultraviolett-Lichtquelle 18 statt eines Xenon-Excimer-Strahlers 22 auch zwei oder mehrerer solcher Strahler in einem oder mehreren parallelen Rohren aufweisen, oder es kann mindestens eine Durchfluss-Excimerlampe verwendet werden, wie sie an sich aus der bereits erwähnten EP 0 697 374 A1 bekannt ist. Es können auch andere Lampentypen wie z. B. Quecksilber(Hg)-Niederdruckdampflampen verwendet werden, die mit elektronischem Vorschaltgerät oder elektrodenlos mit Hochfrequenzanregung (Energieeinkopplung mit Mikrowellengenerator) betrieben werden. Jedoch besteht speziell bei der dielektrisch behinderten Entladung auf der Basis von Xenon-Excimeren der Vorteil in der sofortigen Einsatzbereitschaft gegenüber anderen Entladungslampen, deren Leuchtgas nach der Lampenzündung erst seinen Betriebsdruck erreichen muss. Ein weiterer Vorteil gegenüber Quecksilberdampflampen liegt in der Temperaturunabhängigkeit.

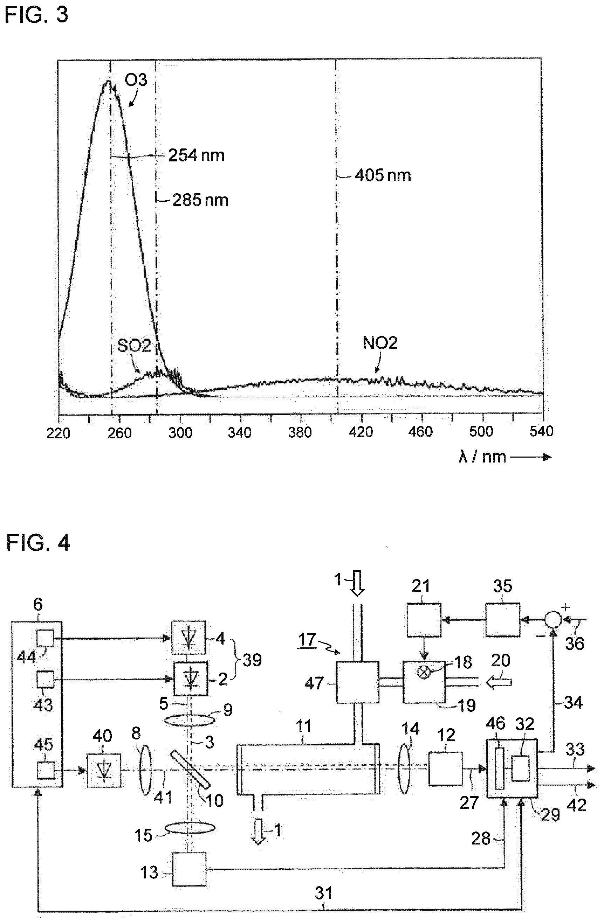

Figur 3 zeigt beispielhaft die Absorptionsspektren von Stickstoffdioxid NO2, Ozon O3 und Schwefeldioxid SO2 sowie die hier durch die Zentralwellenlängen 405 nm und 254 nm repräsentierten Emissionsspektren der beiden Leuchtdioden 2, 4 zur Messung der Komponenten Stickstoffdioxid und Ozon.

Zurück zu Figur 1 erzeugt der das Licht 3, 5 der Leuchtdioden 2, 4 nach Durchstrahlen der Messkammer 11 mit dem behandelten Abgas 1 detektierende Detektor 12 ein Detektorsignal 27, das entsprechend der abwechselnden Ansteuerung der Leuchtdioden 2, 4 zwei abwechselnd aufeinanderfolgende Signalanteile enthält, von denen ein erster Signalanteil aus dem Licht 3 der ersten Leuchtdiode 2 und ein zweiter Signalanteil aus dem Licht der zweiten Leuchtdiode 4 resultiert. Der Referenzdetektor 13 erzeugt ein Referenzsignal 28 mit aus dem Licht 3, 5 beider Leuchtdioden 2, 4 resultierenden und abwechselnd aufeinanderfolgenden Referenz-Signalanteilen. Das Detektorsignal 27 und das Referenzsignal 28 werden einer Auswerteeinrichtung 29 zugeführt, die einen Demultiplexer 30 zur Trennung der unterschiedlichen Signalanteile enthält. Die Synchronisierung des Multiplexers 7 und Demultiplexers 30 erfolgt über eine Kommunikationsleitung 31 zwischen der Steuereinrichtung 6 und der Auswerteeinrichtung 29. Nach Aufbereitung, z. B. Filterung und Digitalisierung, der Signale 27, 28 werden in einer Recheneinrichtung 32 aus dem ersten Signalanteil des Detektorsignals 27 die Stickstoffdioxid-Konzentration und aus dem zweiten Signalanteil des Detektorsignals 27 die überschüssige Ozon-Konzentration des behandelten Abgases 1 in der Messkammer 11 ermittelt. Dabei werden die beiden Signalanteile des Detektorsignals 27 mit den zugehörigen Signalanteilen des Referenzsignals 28 referenziert, so dass die ermittelten Konzentrationen von der Helligkeit der Leuchtdioden 2, 4 und damit z. B. von ihrem Alterungszustand unabhängig sind.

Die ermittelte Stickstoffdioxid-Konzentration in dem behandelten Abgas 1 wird als die Stickoxid-Konzentration 33 des Abgases 1 ausgegeben. Die ermittelte überschüssige Ozon-Konzentration 34 wird als Istwert einer Regeleinrichtung 35 zugeführt, die den Ozongenerator 18, hier das Betriebgsgerät 21 des UV-Strahlers, steuert, um die überschüssige Ozon-Konzentration in dem behandelten Abgas 1 auf einen vorgegebenen Sollwert 36 in Form eines minimalen Rest-Ozongehalts zu regeln. Damit wird sichergestellt, dass nur so viel Ozon produziert wird, wie für die Umsetzung von Stickstoffmonoxid in Stickstoffdioxid erforderlich ist und somit die Umsetzung von Stickstoffmonoxid zu Stickstoffdioxid nahezu vollständig ist.

Wenn der Restsauerstoffgehalt des Abgases 1 oder die Leistung des Ozongenerators 18 für die zur vollständigen Umsetzung von Stickstoffmonoxid in Stickstoffdioxid erforderliche Ozonerzeugung nicht ausreicht, kann über das Dreiwege-Mischventil 16 dem Abgas 1 Luftsauerstoff 20 beigemischt werden. Die Beimischung kann manuell oder automatisch in Abhängigkeit von der ermittelten Ozon-Konzentration 34 in dem behandelten Abgas 1 erfolgen, beispielsweise, wenn die Ozon-Konzentration 34 den Sollwert 36 um ein vorgegebenes Maß unterschreitet. Trotz der erwähnten Referenzierung der Messungen ist es vorteilhaft, die gesamte photometrische Messanordnung des Gasanalysators zu thermostatieren. Dazu gehört auch eine Thermostatisierung der Leuchtdioden 2, 3 mit Hilfe von Peltierelementen 37, 38, um Messbereiche im unteren ppm-Bereich realisieren zu können.

Der gezeigte Gasanalysator kann ohne Weiteres für die Messung weiterer Bestandteile des Abgases 1, wie z. B. Kohlendioxid, Kohlenmonoxid, Schwefel-, Chlor- und Iod-Verbindungen, erweitert werden. Anstelle der dazu erforderlichen weiteren geeigneten Lichtquellen (z. B. Leuchtdioden) können einzelne bereits vorhandene Leuchtdioden mit einem Leuchtstoff (Phosphor) versehen werden, der das von der betreffenden Leuchtdiode erzeugte Licht teilweise in ein Licht mit größerer Wellenlänge umwandelt. Dieses Prinzip ist beispielsweise aus der US 2010/049017 A1 bekannt.

Figur 4 zeigt ein zweites Ausführungsbeispiel des erfindungsgemäßen Gasanalysators das sich von dem nach Figur 1 dadurch unterscheidet, dass die beiden Leuchtdioden 2, 4 (und ggf. weitere, hier nicht gezeigte Leuchtdioden) in einem Array 39 nebeneinander liegend angeordnet sind. Lediglich aus Gründen der besseren Darstellbarkeit sind die Leuchtdioden 2, 4 hier hintereinander liegend dargestellt. Anstelle zweier Kollimatorlinsen für die beiden Leuchtdioden 2, 4 ist hier nur die gemeinsame Kollimatorlinse 9 erforderlich.

Ferner ist zur Messung der Schwefeldioxid-Konzentration in dem Abgas 1 eine dritte Leuchtdiode 40 vorgesehen, die Licht 41 im Wellenlängenbereich zwischen 250 nm und 300 nm, beispielsweise mit einer Zentralwellenlänge von 285 nm, erzeugt.

Figur 3 zeigt beispielhaft das durch die Zentralwellenlänge 285 nm repräsentierte Emissionsspektrum der dritten Leuchtdiode 40.

Das Licht 41 der dritten Leuchtdiode 40 wird mittels der Kollimatorlinse 8 zu einem parallelen Lichtbündel geformt und mittels des Strahlteilers 10 zusammen mit dem Licht 3, 5 der ersten und zweiten Leuchtdiode 2, 4 in einem Teilstrahl durch die Messkammer 11 hindurch auf den Detektor 12 und in einem weiteren Teilstrahl auf den Referenzdetektor 13 geleitet. Das Detektorsignal 27 enthält daher einen aus dem Licht 41 der dritten Leuchtdiode 40 resultierenden dritten Signalanteil, aus dem die Auswerteeinrichtung 29 die Schwefeldioxid-Konzentration 42 des Abgases 1 in der Messkammer 11 ermittelt.

Ein weiterer Unterschied besteht darin, dass die Leuchtdioden 2, 4, 40 nicht im Multiplexbetrieb angesteuert werden, sondern über Signalgeneratoren 43, 44, 45 unterschiedlich moduliert werden, z. B. mit unterschiedlichen Modulationsfrequenzen, Taktraten oder Pulse-Codes. Die Auswerteeinrichtung 29 enthält dementsprechend anstelle eines Demultiplexers einen Demodulator 46, der dazu ausgebildet ist, die Signalanteile des Detektorsignals 27 und des Referenzsignals 28 bei den unterschiedlichen Modulationsfrequenzen oder Taktraten phasensensitiv zu demodulieren oder decodieren, um die Signalanteile für die weitere Verarbeitung und Auswertung zu trennen. Die unterschiedliche Anordnung der Leuchtdioden 2, 4, 40 und ihre unterschiedliche Ansteuerung stellen voneinander unabhängige Maßnahmen dar, die dementsprechend einzeln oder zusammen angewandt werden können.

Schließlich wird das Ozon ausschließlich aus Luftsauerstoff 20 erzeugt und anschließen in einer Mischkammer 47 dem zu behandelnden Abgas 1 beigemischt. Natürlich kann das Ozon auch aus dem Restsauerstoffgehalt des Abgases 1 erzeugt werden, so wie dies in Figur 1 gezeigt ist.

Wie Figur 3 zeigt, besteht eine Querempfindlichkeit der Restkonzentration des Ozons in dem behandelten Abgas 1 auf die Messung der Schwefeldioxid-Konzentration, wobei aber ohne Weiteres eine rechnerische Korrektur des Schwefeldioxid-Konzentrationswertes mit dem Wert der gemessenen Ozon-Restkonzentration möglich ist. Wie die folgenden Beispiele zeigen, kann alternativ die Schwefeldioxid-Konzentration in dem unbehandelten Abgas gemessen werden.

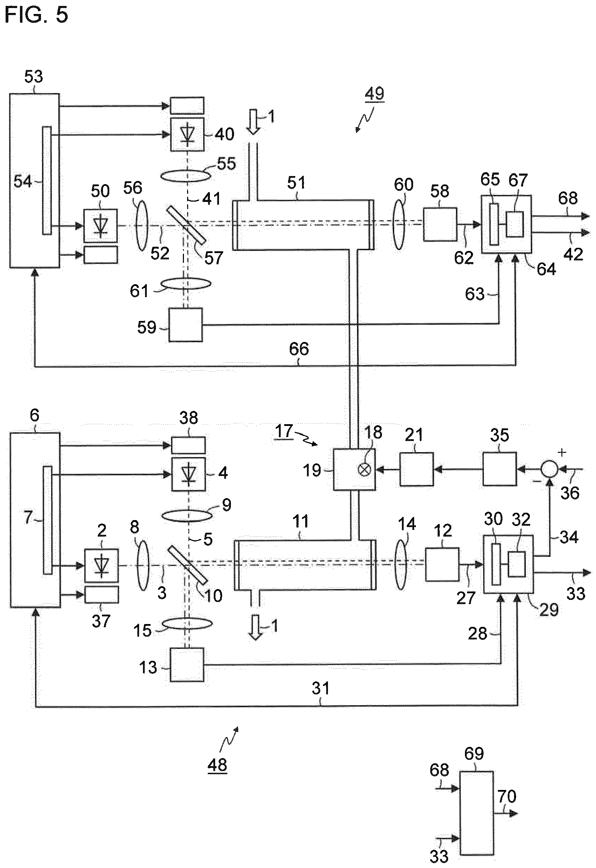

Figur 5 zeigt ein drittes Ausführungsbeispiel des erfindungsgemäßen Gasanalysators, das ebenfalls zur Messung der Stickoxid-Konzentration, Schwefeldioxid-Konzentration und Ozon-Konzentration in dem Abgas 1 dient. Es kommen hier zwei nahezu baugleiche Analysator-Einheiten 48 und 49 zum Einsatz, von denen die Analysator-Einheit 48 dem in Figur 1 gezeigten Gasanalysator mit der ersten und zweiten Leuchtdiode 2, 3, der Messkammer 11 und der Oxidationseinrichtung 17 zur Messung der Stickoxid-Konzentration und Ozon-Konzentration in dem behandelten Abgas 1 entspricht.

Die zweite Analysator-Einheit 49 weist eine dritte Leuchtdiode 40, eine vierte Leuchtdiode 50 und eine weitere Messkammer 51 auf, die von dem unbehandelten Abgas 1 durchströmt wird. Bei dem gezeigten Beispiel strömt das Abgas 1 nacheinander durch die weitere Messkammer 51 der Analysator-Einheit 49, die Oxidationseinrichtung 17 und die Messkammer 11 der Analysator-Einheit 48. Alternativ kann das Abgas 1 in einen Teilstrom durch die die Oxidationseinrichtung 17 und die Messkammer 11 der Analysator-Einheit 48 und einen dazu parallelen weiteren Teilstrom durch die weitere Messkammer 51 der Analysator-Einheit 49 aufgeteilt werden.

Die dritte Leuchtdiode 40 erzeugt Licht 41 im Wellenlängenbereich zwischen 250 nm und 300 nm, beispielsweise mit einer Zentralwellenlänge von 285 nm. Die vierte Leuchtdiode 50 erzeugt Licht 52 in demselben Wellenlängenbereich wie die erste Leuchtdiode 2, also zwischen 350 nm und 500 nm, beispielsweise mit einer Zentralwellenlänge von 405 nm.

Die dritte und vierte Leuchtdiode 40, 50 werden von einer Steuereinrichtung 53 angesteuert, die bei dem hier gezeigten Ausführungsbeispiel einen Multiplexer 54 enthält, um die Leuchtdioden 40, 50 abwechselnd ein- und auszuschalten. Das von den Leuchtdioden 40, 50 abgestrahlte Licht 41, 52 wird mit Hilfe von Kollimatorlinsen 55, 56 zu parallelen Lichtbündeln geformt und mittels eines Polka-Dot-Strahlteilers 57 in einen Teilstrahl durch die weitere Messkammer 51 hindurch auf einen (weiteren) Detektor 58 und einen weiteren Teilstrahl auf einen (weiteren) Referenzdetektor 59 aufgeteilt. Die jeweiligen Teilstrahlen werden mit Hilfe von Linsen 60, 61 auf die Detektoren 58, 59 fokussiert.

Der weitere Detektor 58 erzeugt ein weiteres Detektorsignal 62 mit einem aus dem Licht 41 der dritten Leuchtdiode 40 resultierenden dritten Signalanteil und einem aus dem Licht 52 der vierten Leuchtdiode 50 resultierenden vierten Signalanteil. Der weitere Referenzdetektor 59 erzeugt ein weiteres Referenzsignal 63 mit aus dem Licht 40, 52 der dritten und vierten Leuchtdiode 40, 51 resultierenden dritten und vierten Referenz-Signalanteilen.

Das weitere Detektorsignal 62 und Referenzsignal 63 werden einer weiteren Auswerteeinrichtung 64 zugeführt, die einen Demultiplexer 65 zur Trennung der unterschiedlichen Signalanteile enthält. Die Synchronisierung des Multiplexers 54 und Demultiplexers 65 erfolgt über eine Kommunikationsleitung 66 zwischen der Steuereinrichtung 53 und der Auswerteeinrichtung 64. In einer Recheneinrichtung 67 werden aus dem dritten Signalanteil des Detektorsignals 62 die Schwefeldioxid-Konzentration 42 und aus dem vierten Signalanteil des Detektorsignals 62 die Stickstoffdioxid-Konzentration 68 des unbehandelten Abgases 1 in der Messkammer 51 ermittelt. Dabei werden die beiden Signalanteile des Detektorsignals 62 mit den zugehörigen Signalanteilen des Referenzsignals 63 referenziert.

Die erste Analysator-Einheit 48 ermittelt also die Stickstoffdioxid-Konzentration des UV-behandelten Abgases, welche bei nahezu vollständiger Umsetzung von Stickstoffmonoxid zu Stickstoffdioxid der Stickoxid-Konzentration 33 entspricht. Die zweite Analysator-Einheit 49 ermittelt neben der Schwefeldioxid-Konzentration 42 die Stickstoffdioxid-Konzentration 68 des unbehandelten Abgases 1, so dass in einer Einrichtung 69 durch Bildung der Differenz der von den beiden Auswerteeinrichtungen 29, 64 ermittelten Stickstoffdioxid-Konzentrationen 33, 68 als die Stickstoffmonoxid-Konzentration 70 des Abgases 1 ermittelt und ausgegeben werden kann.

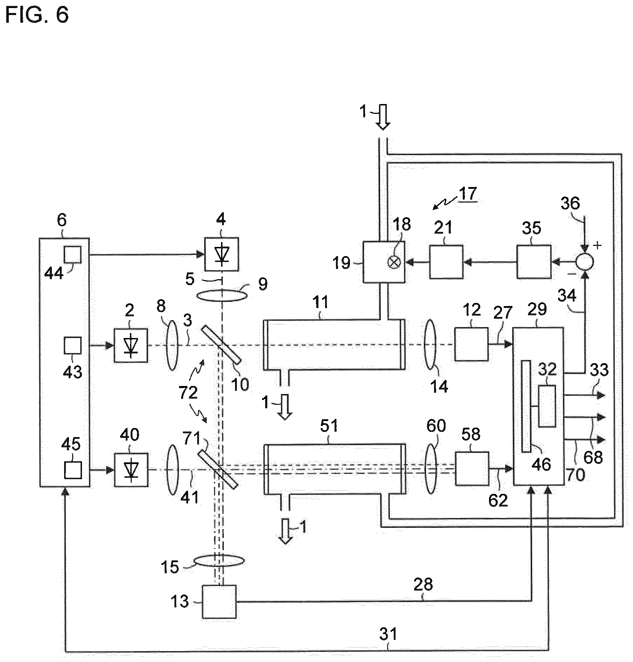

Figur 6 zeigt schließlich ein viertes Ausführungsbeispiel des erfindungsgemäßen Gasanalysators bei dem die erste, zweite und dritte Leuchtdiode 2, 4, 40, die von dem UV-behandelten Abgas 1 durchströmte Messkammer 11 und die von dem unbehandelten Abgas 1 durchströmte weitere Messkammer 51 baulich in einer Analysator-Einheit zusammengefasst sind. Da gezeigtes Beispiel stellt eine Erweiterung des in Figur 4 gezeigten Ausführungsbeispiels um die weitere Messkammer 51 dar. Dabei wird mittels einer hier aus dem Strahlteiler 10 und einem weiteren Strahlteiler 71 bestehenden Strahlteileranordnung 72 ein erster Teil des Lichts 3 der ersten Leuchtdiode 2 durch die von dem behandelten Abgas 1 durchströmte Messkammer 11 auf den Detektor 12 und ein zweiter Teil durch die von dem unbehandelten Abgas 1 durchströmte weitere Messkammer 51 auf den weiteren Detektor 58 abgezweigt. Ein Teil des Lichts 5 der zweiten Leuchtdiode 4 gelangt durch die von dem behandelten Abgas 1 durchströmte Messkammer 11 auf den Detektor 12 und ein Teil des Lichts 41 der dritten Leuchtdiode 40 durch die von dem unbehandelten Abgas 1 durchströmte weitere Messkammer 51 auf den weiteren Detektor 58. Von dem Licht 3, 5, 41 aller Leuchtdioden 2, 4, 40 wird jeweils ein verbleibender Teil auf den Referenzdetektor 13 geleitet.

Wie auch im Beispiel von Figur 4 werden die Leuchtdioden 2, 4, 40 von den Signalgeneratoren 43, 44, 45 der Steuereinrichtung 6 unterschiedlich moduliert. Die Auswerteeinrichtung ist dazu ausgebildet, neben dem Detektorsignal 29 des Detektors 12 auch das weitere Detektorsignal 62 des weiteren Detektors 58 zu verarbeiten und enthält einen Demodulator 46, der die Signalanteile der Detektorsignale 27, 62 und des Referenzsignals 28 demoduliert und für die weitere Verarbeitung und Auswertung in der Recheneinrichtung 32 trennt. Die Recheneinrichtung 32 ermittelt aus dem aus dem Licht 3 der ersten Leuchtdiode 2 resultierenden ersten Signalanteil des Detektorsignals 27 die Stickstoffdioxid-Konzentration des behandelten Abgases 1 und damit die Stickoxid-Konzentration 33. Aus dem aus dem Licht 5 der zweiten Leuchtdiode 2 resultierenden zweiten Signalanteil des Detektorsignals 27 wird die Ozon-Konzentration 34 in dem behandelten Abgas 1 bestimmt. Aus dem aus dem Licht 41 der dritten Leuchtdiode 40 resultierenden dritten Signalanteil in dem weiteren Detektorsignal 62 wird die Schwefeldioxid-Konzentration 42 des Abgases 1 ermittelt. Aus dem aus dem Licht 3 der ersten Leuchtdiode 2 resultierenden vierten Signalanteil des Detektorsignals 27 in dem weiteren Detektorsignal 62 wird die Stickstoffdioxid-Konzentration 68 in dem unbehandelten Abgas 1 bestimmt. Schließlich wird aus der Differenz der in dem behandelten und dem unbehandelten Abgas 1 gemessenen Stickstoffdioxid-Konzentrationen 33, 68 die Stickstoffmonoxid-Konzentration 70 des Abgases 1 ermittelt.

## Patentansprüche

1. Gasanalysator zur Messung von Stickoxiden in einem Abgas (1) mit
- einer Oxidationseinrichtung (17), die einen Ozongenerator (18) zur Erzeugung von Ozon aus Sauerstoff aufweist und zur Behandlung des Abgases (1) mit dem Ozon ausgebildet ist, um in dem Abgas (1) enthaltenes Stickstoffmonoxid in Stickstoffdioxid umzusetzen,
- einer mit einer Zentralwellenlänge zwischen 350 nm und 500 nm strahlenden ersten Leuchtdiode (2),
- einer mit einer Zentralwellenlänge zwischen 250 nm und 265 nm strahlenden zweiten Leuchtdiode (4),
- einer von dem behandelten Abgas (1) durchströmten und dem Licht (3) der ersten Leuchtdiode (2) und dem Licht (5) der zweiten Leuchtdiode (4) durchstrahlten Messkammer (11),
- einem das Licht (3) der ersten Leuchtdiode (2) und das Licht (5) der zweiten Leuchtdiode (4) nach Durchstrahlen der Messkammer (11) detektierenden Detektor (12), welcher ein Detektorsignal (27) mit einem aus dem Licht (3) der ersten Leuchtdiode (2) resultierenden ersten Signalanteil und einen aus dem Licht (5) der zweiten Leuchtdiode (4) resultierenden zweiten Signalanteil erzeugt,
- einer Auswerteeinrichtung (29), die aus dem ersten Signalanteil die Stickstoffdioxid-Konzentration des behandelten Abgases (1) in der Messkammer (11) ermittelt und als die Stickoxid-Konzentration (33) des Abgases (1) ausgibt und aus dem zweiten Signalanteil die Ozon-Konzentration des Abgases (1) in der Messkammer (11) ermittelt,
**dadurch gekennzeichnet,**
- **dass** der Ozongenerator (18) regelbar ist, und
- **dass** eine den Ozongenerator (18) steuernde Regeleinrichtung (35) vorhanden ist, die die ermittelte Ozon-Konzentration als Istwert (34) erhält, um sie auf einen vorgegebenen Sollwert (36) zu regeln.

2. Gasanalysator nach Anspruch 2, **dadurch gekennzeichnet, dass** ein Strahlteiler (10) vorgesehen ist, der einen Teil des Lichts (3, 5) der ersten und zweiten Leuchtdiode (2, 4) auf einen Referenzdetektor (13) abzweigt, dass der Referenzdetektor (13) ein Referenzsignal (28) mit aus dem Licht (3, 5) der ersten und zweiten Leuchtdiode (2, 4) resultierenden ersten und zweiten Referenz-Signalanteilen erzeugt und dass die Auswerteeinrichtung (29) die Signalanteile des Detektorsignals (27) mit den zugehörigen Referenz-Signalanteilen referenziert.

3. Gasanalysator nach Anspruch 1 oder 2, **dadurch gekennzeichnet, dass** der Ozongenerator (18) eine Ultraviolett-Lichtquelle aufweist.

4. Gasanalysator nach Anspruch 3, **dadurch gekennzeichnet, dass** die Ultraviolett-Lichtquelle (18) mindestens einen Xenon-Excimer-Strahler (22) aufweist.

5. Gasanalysator nach Anspruch 3 oder 4, **dadurch gekennzeichnet, dass** die Ultraviolett-Lichtquelle (18) in einer von dem Abgas (1) durchströmten geschlossenen Reaktionskammer (19) angeordnet und dazu ausgebildet ist, das Ozon aus dem Restsauerstoffgehalt des Abgases (1) zu erzeugen.

6. Gasanalysator nach einem der vorangehenden Ansprüche, **dadurch gekennzeichnet,**
- **dass** eine mit einer Zentralwellenlänge zwischen 250 nm und 300 nm strahlende dritte Leuchtdiode (40) vorhanden ist, deren Licht (41) ebenfalls die Messkammer (11) durchstrahlt und anschließend von dem Detektor (12) detektiert wird, so dass das Detektorsignal (27) einen aus dem Licht (41) der dritten Leuchtdiode (40) resultierenden dritten Signalanteil enthält, und
- **dass** die Auswerteeinrichtung (29) aus dem dritten Signalanteil die Schwefeldioxid-Konzentration des Abgases (1) in der Messkammer (11) ermittelt.

7. Gasanalysator nach Anspruch 6 in Verbindung mit Anspruch 2, **dadurch gekennzeichnet, dass** der Strahlteiler (10) einen Teil des Lichts (41) der dritten Leuchtdiode (40) auf den Referenzdetektor (13) abzweigt, so dass das Referenzsignal (28) einen aus dem Licht (41) der dritten Leuchtdiode (40) resultierenden dritten Referenz-Signalanteil enthält, und dass die Auswerteeinrichtung (29) den dritten Signalanteil des Detektorsignals (27) mit dem dritten Referenz-Signalanteil referenziert.

8. Gasanalysator nach einem der Ansprüche 1 bis 5, mit
- einer mit einer Zentralwellenlänge zwischen 250 nm und 300 nm strahlende dritten Leuchtdiode (40),
- einer mit einer Zentralwellenlänge zwischen 350 nm und 500 nm, vorzugsweise mit derselben Zentralwellenlänge wie die erste Leuchtdiode (2) strahlenden vierten Leuchtdiode (50),
- einer von dem unbehandelten Abgas (1) durchströmten und dem Licht (41, 52) der dritten und vierten Leuchtdiode (40, 50) durchstrahlten weiteren Messkammer (51),
- einem das Licht (41, 52) der dritten und vierten Leuchtdiode (40, 50) nach Durchstrahlen der weiteren Messkammer (51) detektierenden weiteren Detektor (58), welcher ein weiteres Detektorsignal (62) mit einem aus dem Licht (41) der dritten Leuchtdiode (40) resultierenden dritten Signalanteil und einem aus dem Licht (52) der vierten Leuchtdiode (50) resultierenden vierten Signalanteil erzeugt,
- einer weiteren Auswerteeinrichtung (64), die aus dem dritten Signalanteil die Schwefeldioxid-Konzentration und aus dem vierten Signalanteil die Stickstoffdioxid-Konzentration (68) des unbehandelten Abgases in der weiteren Messkammer (51) ermittelt, und
- einer Einrichtung (69), die die Differenz der von den beiden Auswerteeinrichtungen (29, 64) ermittelten Stickstoffdioxid-Konzentrationen (33, 68) als Stickstoffmonoxid-Konzentration (70) des Abgases (1) ausgibt (Figur 5).

9. Gasanalysator nach Anspruch 8, **dadurch gekennzeichnet, dass** ein weiterer Strahlteiler (57) vorgesehen ist, der einen Teil des Lichts (41, 52) der dritten und vierten Leuchtdiode (40, 50) auf einen weiteren Referenzdetektor (59) abzweigt, dass der weitere Referenzdetektor (59) ein weiteres Referenzsignal (63) mit aus dem Licht (40, 52) der dritten und vierten Leuchtdiode (40, 50) resultierenden dritten und vierten Referenz-Signalanteilen erzeugt und dass die weitere Auswerteeinrichtung (64) die dritten und vierten Signalanteile des weiteren Detektorsignals (62) mit den zugehörigen Referenz-Signalanteilen referenziert.

10. Gasanalysator nach einem der Ansprüche 1 bis 5, mit
- einer mit einer Zentralwellenlänge zwischen 250 nm und 300 nm strahlende dritten Leuchtdiode (40),
- einer von dem unbehandelten Abgas (1) durchströmten und dem Licht (41) der dritten Leuchtdiode (40) durchstrahlten weiteren Messkammer (51),
- einer Strahlteileranordnung (72), die einen Teil des Lichts (3) der ersten Leuchtdiode (2) durch die weitere Messkammer (51) leitet,
- einem das Licht (3, 41) der ersten und dritten Leuchtdiode (2, 40) nach Durchstrahlen der weiteren Messkammer (51) detektierenden weiteren Detektor (58), welcher ein weiteres Detektorsignal (62) mit einem aus dem Licht (41) der dritten Leuchtdiode (40) resultierenden dritten Signalanteil und einem aus dem Licht (3) der ersten Leuchtdiode (2) resultierenden vierten Signalanteil erzeugt, und
- dass die Auswerteeinrichtung (29) ferner aus dem dritten Signalanteil die Schwefeldioxid-Konzentration und aus dem vierten Signalanteil die Stickstoffdioxid-Konzentration des unbehandelten Abgases (1) in der weiteren Messkammer (51) ermittelt und die Differenz der aus dem ersten und vierten Signalanteil ermittelten Stickstoffdioxid-Konzentrationen (33, 68) als Stickstoffmonoxid-Konzentration (70) des Abgases (1) ausgibt (Figur 6).

11. Gasanalysator nach Anspruch 10, **dadurch gekennzeichnet, dass** die Strahlteileranordnung (72) dazu ausgebildet ist, einen Teil des Lichts (3, 5, 41) der ersten, zweiten und dritten Leuchtdiode (2, 4, 40) auf einen Referenzdetektor (13) abzuzweigen, der ein Referenzsignal (28) mit aus dem Licht (3, 5, 41) der Leuchtdioden (2, 4, 40) resultierenden Referenz-Signalanteilen erzeugt und dass die Auswerteeinrichtung (29) die Signalanteile der Detektorsignale (27, 62) mit den zugehörigen Referenz-Signalanteilen referenziert.

12. Verfahren zur Messung von Stickoxiden in einem Abgas (1) wobei
- mittels eines Ozongenerators (18) aus Sauerstoff Ozon erzeugt wird,
- das Abgas (1) mit dem erzeugten Ozon behandelt wird, um in dem Abgas (1) enthaltenes Stickstoffmonoxid in Stickstoffdioxid umzusetzen, und
- die Stickstoffdioxid-Konzentration des behandelten Abgases (1) photometrisch unter Verwendung einer mit einer Zentralwellenlänge zwischen 350 nm und 500 nm strahlenden ersten Leuchtdiode (2) gemessen und als die Stickoxid-Konzentration (33) des Abgases (1) ausgegeben wird und
- die Ozon-Konzentration des behandelten Abgases (1) photometrisch unter Verwendung einer mit einer Zentralwellenlänge zwischen 250 nm und 265 nm strahlenden zweiten Leuchtdiode (4) gemessen wird,
- wobei das Licht der ersten Leuchtdiode (2) und der zweiten Leuchtdiode (4) eine mit dem behandelten Abgas (1) durchströmte Messkammer (11) durchstrahlt,
**dadurch gekennzeichnet,**
- **dass** der Ozongenerator (18) regelbar ist und mit einer Regeleinrichtung (35) gesteuert wird, und
- **dass** die Regeleinrichtung (35) die gemessene Ozon-Konzentration als Ist-Wert erhält und sie auf einen vorgegebenen Sollwert regelt.

13. Verfahren nach Anspruch 12, **dadurch gekennzeichnet, dass** die Schwefeldioxid-Konzentration in dem behandelten oder unbehandelten Abgas (1) photometrisch unter Verwendung einer mit einer Zentralwellenlänge zwischen 250 nm und 300 nm strahlenden dritten Leuchtdiode (40) gemessen wird.

14. Verfahren nach Anspruch 12 oder 13, **dadurch gekennzeichnet, dass** die Stickstoffdioxid-Konzentration in dem unbehandelten Abgas (1) photometrisch unter Verwendung einer mit einer Zentralwellenlänge zwischen 350 nm und 500 nm, vorzugsweise mit derselben Zentralwellenlänge wie die erste Leuchtdiode (2) strahlenden vierten Leuchtdiode (50) gemessen wird und aus der Differenz der in dem behandelten Abgas (1) und in dem unbehandelten Abgas (1) ermittelten Stickstoffdioxid-Konzentrationen die Stickstoffmonoxid-Konzentration des Abgases (1) ermittelt wird.

## Claims

1. Gas analyser for measuring nitrogen oxides in an exhaust gas (1) with
- an oxidation device (17) comprising an ozone generator (18) for the generation of ozone from oxygen and embodied to treat the exhaust gas (1) with the ozone in order to convert nitrogen monoxide contained in the exhaust gas (1) into nitrogen dioxide,
- a first light-emitting diode (2) that emits with a central wavelength of between 350 nm and 500 nm,
- a second light-emitting diode (4) that emits with a central wavelength of between 250 nm and 265 nm,
- a measuring chamber (11) through which the treated exhaust gas (1) flows and the light (3) from the first light-emitting diode (2) and the light (5) from the second light-emitting diode (4) pass,
- a detector (12) that detects the light (3) from the first light-emitting diode (2) and the light (5) from the second light-emitting diode (4) after it has passed through the measuring chamber (11) and which generates a detector signal (27) with a first signal component resulting from the light (3) from the first light-emitting diode (2) and a second signal component resulting from the light (5) from the second light-emitting diode (4),
- an evaluation device (29), which ascertains the nitrogen dioxide concentration in the treated exhaust gas (1) in the measuring chamber (11) from the first signal component and outputs this as the nitrogen oxide concentration (33) in the exhaust gas (1) and ascertains the ozone concentration in the exhaust gas (1) in the measuring chamber (11) from the second signal component,
**characterised in that**
- the ozone generator (18) can be regulated, and
- a regulating device (35) that controls the ozone generator (18) is provided, which obtains the ascertained ozone concentration as an actual value (34) in order to regulate this to a prespecified setpoint value (36).

2. Gas analyser according to claim 2, **characterised in that** a beam splitter (10) is provided and diverts a part of the light (3, 5) from the first and second light-emitting diode (2, 4) to a reference detector (13), that the reference detector (13) generates a reference signal (28) with first and second reference-signal components resulting from the light (3, 5) from the first and second light-emitting diode (2, 4) and that the evaluation device (29) references the signal components of the detector signal (27) with the associated reference-signal components.

3. Gas analyser according to claim 1 or 2, **characterised in that** the ozone generator (18) comprises an ultraviolet light source.

4. Gas analyser according to claim 3, **characterised in that** the ultraviolet light source (18) comprises at least one xenon excimer emitter (22).

5. Gas analyser according to claim 3 or 4, **characterised in that** the ultraviolet light source (18) is arranged in a closed reaction chamber (19) through which the exhaust gas (1) flows and which is embodied to generate the ozone from the residual oxygen content of the exhaust gas (1).

6. Gas analyser according to one of the preceding claims, **characterised in**
- **that** a third light-emitting diode (40) that emits with a central wavelength of between 250 nm and 300 nm is provided, the light (41) of which also passes through the measuring chamber (11) and is then detected by the detector (12) so that the detector signal (27) contains a third signal component resulting from the light (41) from the third light-emitting diode (40) and
- **that** the evaluation device (29) ascertains the sulfur dioxide concentration in the exhaust gas (1) in the measuring chamber (11) from the third signal component.

7. Gas analyser according to claim 6 in conjunction with claim 2, **characterised in that** the beam splitter (10) diverts a part of the light (41) from the third light-emitting diode (40) to the reference detector (13) so that the reference signal (28) contains a third reference-signal component resulting from the light (41) from the third light-emitting diode (40) and that the evaluation device (29) references the third signal component of the detector signal (27) with the third reference-signal component.

8. Gas analyser according to one of claims 1 to 5, with
- a third light-emitting diode (40) that emits with a central wavelength of between 250 nm and 300 nm,
- a fourth light-emitting diode (50) that emits with a central wavelength of between 350 nm and 500 nm, preferably with the same central wavelength as the first light-emitting diode (2),
- a further measuring chamber (51) through which the untreated exhaust gas (1) flows and the light (41, 52) from the third and fourth light-emitting diode (40, 50) passes,
- a further detector (58) that detects the light (41, 52) from the third and fourth light-emitting diode (40, 50) after it has passed through the further measuring chamber (51) and which generates a further detector signal (62) with a third signal component resulting from the light (41) from the third light-emitting diode (40) and a fourth signal component resulting from the light (52) from the fourth light-emitting diode (50),
- a further evaluation device (64) that ascertains the sulfur dioxide concentration in the untreated exhaust gas in the further measuring chamber (51) from the third signal component and nitrogen dioxide concentration (68) from the fourth signal component and
- a device (69) that outputs the difference between the nitrogen dioxide concentrations (33, 68) ascertained by the two evaluation devices (29, 64) as the nitrogen monoxide concentration (70) in the exhaust gas (1) (Figure 5).

9. Gas analyser according to claim 8, **characterised in that** a further beam splitter (57) is provided and diverts a part of the light (41, 52) from the third and fourth light-emitting diode (40, 50) to a further reference detector (59), that the further reference detector (59) generates a further reference signal (63) with third and fourth reference-signal components resulting from the light (40, 52) from the third and fourth light-emitting diode (40, 50) and that the further evaluation device (64) references the third and fourth signal components of the further detector signal (62) with the associated reference-signal components.

10. Gas analyser according to one of claims 1 to 5, with
- a third light-emitting diode (40) that emits with a central wavelength of between 250 nm and 300 nm,
- a further measuring chamber (51) through which the untreated exhaust gas (1) flows and the light (41) from the third light-emitting diode (40) passes,
- a beam-splitter arrangement (72) that conducts a part of the light (3) from the first light-emitting diode (2) through the further measuring chamber (51),
- a further detector (58) that detects the light (3, 41) from the first and third light-emitting diode (2, 40) after it has passed through the further measuring chamber (51) and which generates a further detector signal (62) with a third signal component resulting from the light (41) from the third light-emitting diode (40) and a fourth signal component resulting from the light (3) from the first light-emitting diode (2) and
- that the evaluation device (29) furthermore ascertains the sulfur dioxide concentration in the untreated exhaust gas (1) in the further measuring chamber (51) from the third signal component and the nitrogen dioxide concentration from the fourth signal component and outputs the difference between the nitrogen dioxide concentrations (33, 68) ascertained from the first and fourth signal component as the nitrogen monoxide concentration (70) in the exhaust gas (1) (Figure 6).

11. Gas analyser according to claim 10, **characterised in that** the beam-splitter arrangement (72) is embodied to divert a part of the light (3, 5, 41) from the first, second and third light-emitting diode (2, 4, 40) to a reference detector (13), which generates a reference signal (28) with reference-signal components resulting from the light (3, 5, 41) from the light-emitting diodes (2, 4, 40) and that the evaluation device (29) references the signal components of the detector signals (27, 62) with the associated reference-signal components.

12. Method for measuring nitrogen oxides in an exhaust gas (1), wherein
- ozone is generated from oxygen by means of an ozone generator (18),
- the exhaust gas (1) is treated with the ozone generated in order to convert nitrogen monoxide contained in the exhaust gas (1) into nitrogen dioxide and
- the nitrogen dioxide concentration in the treated exhaust gas (1) is measured photometrically using a first light-emitting diode (2) that emits with a central wavelength of between 350 nm and 500 nm and output as the nitrogen oxide concentration (33) in the exhaust gas (1) and
- the ozone concentration in the treated exhaust gas (1) is measured photometrically using a second light-emitting diode (4) that emits with a central wavelength of between 250 nm and 265 nm,
- wherein the light from the first light-emitting diode (2) and the second light-emitting diode (4) passes through a measuring chamber (11) through which the treated exhaust gas (1) flows,
**characterised in that**
- the ozone generator (18) can be regulated and is controlled by a regulating device (35) and
- the regulating device (35) obtains the measured ozone concentration as an actual value and regulates it to a prespecified setpoint value.

13. Method according to claim 12, **characterised in that** the sulfur dioxide concentration in the treated or untreated exhaust gas (1) is measured photometrically using a third light-emitting diode (40) that emits with a central wavelength of between 250 nm and 300 nm.

14. Method according to claim 12 or 13, **characterised in that** the nitrogen dioxide concentration in the untreated exhaust gas (1) is measured photometrically using a fourth light-emitting diode (50) that emits with a central wavelength of between 350 nm and 500 nm, preferably with the same central wavelength as the first light-emitting diode (2) and the nitrogen monoxide concentration in the exhaust gas (1) is ascertained from the difference between the nitrogen dioxide concentrations ascertained in the treated exhaust gas (1) and in the untreated exhaust gas (1).

## Revendications

1. Analyseur de gaz pour la mesure d'oxydes d'azote dans un gaz (1) d'échappement, comprenant
- un dispositif (17) d'oxydation, qui a un générateur (18) d'ozone pour la production d'ozone à partir d'oxygène et qui est constitué pour le traitement du gaz (1) d'échappement par l'ozone afin de transformer le monoxyde d'azote contenu dans le gaz (1) d'échappement en dioxyde d'azote,
- une première diode (2) électroluminescente rayonnant à une longueur d'onde centrale comprise entre 350 nm et 500 nm,
- une deuxième diode (4) électroluminescente rayonnant à une longueur d'onde centrale comprise entre 250 nm et 265 nm,
- une chambre (11) de mesure dans laquelle passent le gaz (1) d'échappement à traiter et la lumière (3) de la première diode (2) électroluminescente et la lumière (5) de la deuxième diode (4) électroluminescente,
- un détecteur (12) détectant la lumière (3) de la première diode (2) électroluminescente et la lumière (5) de la deuxième diode (4) électroluminescente après passage dans la chambre (11) de mesure et produisant un signal (27) de détecteur ayant une première partie de signal provenant de la lumière (3) de la première diode (2) électroluminescente et une deuxième partie de signal provenant de la lumière (5) de la deuxième diode (4) électroluminescente,
- un dispositif (29) d'exploitation, qui, à partir de la première partie du signal, détermine la concentration en dioxyde d'azote du gaz (1) d'échappement traité dans la chambre (11) de mesure et la donne comme la concentration (33) en oxyde d'azote du gaz (1) d'échappement et qui, à partir de la deuxième partie de signal, détermine la concentration en ozone du gaz (1) d'échappement dans la chambre (11) de mesure,
**caractérisé**
- **en ce que** le générateur (18) d'ozone est réglable, et
- **en ce qu'**il y a un dispositif (35) de réglage, qui commande le générateur (18) d'ozone et qui reçoit la concentration en ozone déterminée comme valeur (34) réelle pour la régler à une valeur (36) de consigne donnée à l'avance.

2. Analyseur de gaz suivant la revendication 1, **caractérisé en ce qu'**il est prévu un diviseur (10) de faisceau, qui dérive une partie de la lumière (3, 5) des première et deuxième diodes (2, 4) électroluminescentes sur un détecteur (13) de référence, **en ce que** le détecteur (13) de référence produit un signal (28) de référence ayant des première et deuxième parties de signal de référence provenant de la lumière (3, 5) des première et deuxième diodes (2, 4) électroluminescentes et **en ce que** le dispositif (29) d'exploitation établit un rapport entre les parties du signal (27) du détecteur et les parties de signal de référence associées.

3. Analyseur de gaz suivant la revendication 1 ou 2, **caractérisé en ce que** le générateur (18) d'ozone a une source de lumière ultraviolette.

4. Analyseur de gaz suivant la revendication 3, **caractérisé en ce que** la source (18) de lumière ultraviolette a au moins un émetteur (22) à excimère de xénon.

5. Analyseur de gaz suivant la revendication 3 ou 4, **caractérisé en ce que** la source (18) de lumière ultraviolette est disposée dans une chambre (19) de réaction fermée et dans laquelle passe le gaz (1) d'échappement et est constituée pour produire l'ozone à partir de la teneur résiduelle en oxygène du gaz (1) d'échappement.

6. Analyseur de gaz suivant l'une des revendications précédentes, **caractérisé**
- **en ce qu'**il y a une troisième diode (40) électroluminescente rayonnant à une longueur d'onde centrale comprise entre 250 nm et 300 nm, dont la lumière (41) passe également dans la chambre (11) de mesure et est détectée ensuite par le détecteur (12), de sorte que le signal (27) du détecteur contient une troisième partie de signal provenant de la lumière (41) de la troisième diode (40) électroluminescente, et
- **en ce que** le dispositif (29) d'exploitation détermine, à partir de la troisième partie de signal, la concentration en dioxyde de soufre du gaz (1) d'échappement dans la chambre (11) de mesure.

7. Analyseur de gaz suivant la revendication 6 en liaison avec la revendication 2, **caractérisé en ce que** le diviseur (10) de faisceau dérive une partie de la lumière (41) de la troisième diode (40) électroluminescente, de manière à ce que le signal (28) de référence contienne une troisième partie de signal de référence provenant de la lumière (41) de la troisième diode (40) électroluminescente, et **en ce que** le dispositif (29) d'exploitation établit un rapport entre la troisième partie du signal (27) du détecteur et la troisième partie de signal de référence.

8. Analyseur de gaz suivant l'une des revendications 1 à 5, comprenant
- une troisième diode (40) électroluminescente rayonnant à une longueur d'onde centrale comprise entre 250 nm et 300 nm,
- une quatrième diode (50) électroluminescente rayonnant à une longueur d'onde centrale comprise entre 350 nm et 500 nm, de préférence à la même longueur d'onde centrale que la première diode (2) électroluminescente,
- une autre chambre (51) de mesure dans laquelle passent le gaz (1) d'échappement non-traité et la lumière (41, 52) des troisième et quatrième diodes (40, 50) électroluminescentes,
- un autre détecteur (58), qui détecte la lumière (41, 52) des troisième et quatrième diodes (40, 50) électroluminescentes, après traversée de l'autre chambre (51) de mesure, et qui produit un autre signal (62) de détecteur ayant une troisième partie de signal provenant de la lumière (41) de la troisième diode (40) électroluminescente et une quatrième partie de signal provenant de la lumière (52) de la quatrième diode (50) électroluminescente,
- un autre dispositif (64) d'exploitation, qui, à partir de la troisième partie de signal, détermine la concentration en dioxyde de soufre et, à partir de la quatrième partie de signal, détermine la concentration (68) en dioxyde d'azote du gaz (1) d'échappement non-traité dans l'autre chambre (51) de mesure, et
- un dispositif (69), qui donne la différence entre les concentrations (33, 68) en dioxyde d'azote déterminées par les deux dispositifs (29, 64) d'exploitation comme concentration (70) en monoxyde d'azote du gaz (1) d'échappement (figure 5).

9. Analyseur de gaz suivant la revendication 8, **caractérisé en ce qu'**il est prévu un autre diviseur (57) de faisceau, qui dérive une partie de la lumière (41, 52) des troisième et quatrième diodes (40, 50) électroluminescentes sur un autre détecteur (59) de référence, **en ce que** l'autre détecteur (59) de référence produit un autre signal (63) de référence ayant des troisième et quatrième parties de signal de référence provenant de la lumière des troisième et quatrième diodes (40, 50) électroluminescentes et **en ce que** l'autre dispositif (64) d'exploitation établit un rapport entre les troisième et quatrième parties de l'autre signal (62) de détecteur et les parties de signal de référence associées.

10. Analyseur de gaz suivant l'une des revendications 1 à 5, comprenant
- une troisième diode (40) électroluminescente rayonnant à une longueur d'onde centrale comprise entre 250 nm et 300 nm,
- une autre chambre (51) de mesure dans laquelle passent le gaz (1) d'échappement non-traité et la lumière (41) de la troisième diode (40) électroluminescente,
- un agencement (72) de diviseur de faisceau, qui fait passer une partie de la lumière (3) de la première diode (2) électroluminescente dans l'autre chambre (51) de mesure,
- un autre détecteur (58), qui détecte la lumière (3, 41) des première et troisième diodes (2, 40) électroluminescentes, après passage dans l'autre chambre (51) de mesure, et qui produit un autre signal (62) de détecteur ayant une troisième partie de signal provenant de la lumière (41) de la troisième diode (40) électroluminescente et une quatrième partie de signal provenant de la lumière (3) de la première diode (2) électroluminescente, et
- en ce que le dispositif (29) d'exploitation détermine, en outre, à partir de la troisième partie de signal, la concentration en dioxyde de soufre et, à partir de la quatrième partie de signal, la concentration en dioxyde d'azote du gaz (1) d'échappement non-traité dans l'autre chambre (51) de mesure, et donne la différence des concentrations (33, 68) de dioxyde d'azote déterminées à partir des première et quatrième parties de signal comme concentration (70) en monoxyde d'azote du gaz (1) d'échappement (figure 6).

11. Analyseur de gaz suivant la revendication 10, **caractérisé en ce que** l'agencement (72) de diviseur de faisceau est constitué pour dériver une partie de la lumière (3, 5, 41) des première, deuxième et troisième diodes (2,4, 40) électroluminescentes sur un détecteur (13) de référence, qui produit un signal (28) de référence ayant des parties de signal de référence provenant de la lumière (3, 5, 41) des diodes (2, 4, 40) électroluminescentes et **en ce que** le dispositif (29) d'exploitation établit un rapport entre les parties des signaux (27, 62) du détecteur et les parties de signal de référence associées.

12. Procédé de mesure d'oxydes d'azote dans un gaz (1) d'échappement, dans lequel
- on produit de l'ozone à partir de l'oxygène au moyen d'un générateur (18) d'ozone,
- on traite le gaz (1) d'échappement par l'ozone produite, afin de transformer le monoxyde d'azote contenu dans le gaz (1) d'échappement en du dioxyde d'azote, et
- on mesure la concentration en dioxyde d'azote du gaz (1) d'échappement traité, photométriquement en utilisant une première diode (2) électroluminescente rayonnant à une longueur d'onde centrale comprise entre 350 nm et 500 nm et on la donne comme la concentration (33) en oxyde d'azote du gaz (1) d'échappement, et
- on mesure la concentration en ozone du gaz (1) d'échappement traité, photométriquement en utilisant une deuxième diode (4) électroluminescente rayonnant à une longueur d'onde centrale comprise entre 250 nm et 265 nm,
- la lumière de la première diode (2) électroluminescente et de la deuxième diode (4) électroluminescente passant dans une chambre (11) de mesure, dans laquelle passe le gaz (1) d'échappement traité,
**caractérisé**
- **en ce que** le générateur (18) d'ozone est réglable et est commandé par un dispositif (35) de réglage, et
- **en ce que** le dispositif (35) de réglage reçoit la concentration en ozone mesurée comme valeur réelle et la règle à une valeur de consigne donnée à l'avance.

13. Procédé suivant la revendication 12, **caractérisé en ce que** l'on mesure la concentration en dioxyde de soufre du gaz (1) d'échappement traité ou non-traité, photométriquement en utilisant une troisième diode (40) électroluminescente rayonnant à une longueur d'onde centrale comprise entre 250 nm et 300 nm.

14. Procédé suivant la revendication 12 ou 13, **caractérisé en ce que** l'on mesure la concentration en dioxyde d'azote du gaz (1) d'échappement non-traité, photométriquement en utilisant une quatrième diode (50) électroluminescente rayonnant à une longueur d'onde centrale comprise entre 350 nm et 500 nm, de préférence à la même longueur d'onde centrale que la première diode (2) électroluminescente, et on détermine, à partir de la différence des concentrations en dioxyde d'azote déterminées dans le gaz (1) d'échappement traité et dans le gaz (1) d'échappement non-traité, la concentration en monoxyde d'azote du gaz (1) d'échappement.
